# FASCICULE DE BREVET EUROPEEN

(11) **EP 0 404 648 B1**
(45) Date de publication et mention de la délivrance du brevet: **02.02.1994**
(21) Numéro de dépôt: 90401692.0
(22) Date de dépôt: 15.06.1990
(51) Int. Cl.: A61F 13/15

(54) **Couche-culotte à ceinture élastique**
Windel mit einem elastischen Taillenbereich
Diaper with elastic waist band

(30) Priorité: 15.06.1989 FR 8907975
(43) Date de publication de la demande: 27.12.1990
(73) Titulaire: PEAUDOUCE, 59126 Linselles (FR)
(72) Inventeur: Leroy, André, F-59420 Mouvaux (FR)
(74) Mandataire: Casalonga, Axel

(56) Documents cités:
- EP-A- 0 211 197
- EP-A- 0 352 208
- WO-A-88/00442
- US-A- 4 685 916
- US-A- 4 801 485

## Description

La présente invention se rapporte à une couche-culotte comprenant une feuille extérieure imperméable aux liquides, une feuille intérieure perméable aux liquides, les deux feuilles ayant une forme générale sensiblement rectangulaire, un coussin absorbant disposé entre lesdites deux feuilles, le coussin ayant des dimensions inférieures à celles des deux feuilles et étant disposé de manière que ses bords se trouvent en retrait par rapport aux bords correspondants des deux feuilles, lesdites deux feuilles étant reliées entre elles par collage le long de leurs bords, autour du coussin absorbant, des attaches adhésives disposées sur les deux bords longitudinaux opposés des feuilles, au voisinage de l'un des deux bords transversaux opposés desdites feuilles, et un moyen élastique de ceinture s'étendant à l'état tendu le long de l'un au moins des deux bords transversaux desdites feuilles.

Sur les couches-culottes connues de ce type (voir par exemple les documents EP-A-112655, EP-A-119825, EP-A-119827 et FR-A-2 587 175), les moyens élastiques de ceinture sont généralement fixés à la fois à la feuille extérieure imperméable et à la feuille intérieure perméable. Par conséquent, ces moyens élastiques doivent être situés entre un bord transversal du coussin absorbant et le bord transversal correspondant des deux feuilles. Or, sur la plupart des couches-culottes, les attaches adhésives qui servent à fermer la couche-culotte autour de la taille se trouvent davantage en retrait par rapport aux bords transversaux des feuilles que les bords transversaux correspondants du coussin absorbant. C'est la raison pour laquelle, sur ces couches-culottes connues, les moyens élastiques ne sont pas en alignement avec les attaches adhésives.

Or, la fonction principale des moyens élastiques de ceinture sur les couches-culottes est de procurer une certaine élasticité au niveau des attaches adhésives, afin de compenser au maximum les variations de tour de taille qui se produisent, notamment sur des enfants en bas âge, pendant la prise des repas, et pour compenser également les imprécisions susceptibles d'intervenir lors du positionnement des attaches adhésives au moment de la fermeture des couches-culottes.

Par conséquent, sur les couches-culottes connues, les moyens élastiques de ceinture, du fait de leur position, ne remplissent pas ou ne remplissent que mal leur fonction.

Par ailleurs, les moyens élastiques fixés à la feuille extérieure imperméable provoquent une contraction de cette dernière, ce qui donne souvent à la couche-culotte un aspect peu plaisant et peut nuire à la fonction d'étanchéité en raison du fronçage que subit ainsi la feuille extérieure imperméable.

Par le brevet US n° 4 685 916, il est connu de prévoir, sur une couche-culotte, des moyens élastiques de ceinture constitués par des rubans élastiques disposés entre les deux feuilles de la couche-culotte et fixés, soit à la feuille extérieure, soit à la feuille intérieure, soit aux deux feuilles. Ces rubans élastiques présentent une longueur inférieure à la moitié de la largeur de la couche-culotte à l'endroit des attaches adhésives.

Même en cas de fixation de ces rubans élastiques uniquement sur la feuille intérieure de la couche-culotte, il subsiste une liaison relativement étroite des moyens élastiques de ceinture avec la partie restante de la couche-culotte, alors qu'en vue d'obtenir un meilleur ajustement de la couche-culotte sur le corps, il serait désirable que les moyens élastiques de ceinture aient une liberté maximale par rapport à la partie restante de la couche-culotte. De plus, la longueur réduite de ces rubans élastiques limite la course élastique des moyens élastiques de ceinture, alors que pour améliorer le confort et les possibilités d'ajustement, il serait désirable que cette course élastique soit la plus grande possible.

La présente invention a pour objet une couche-culotte remédiant aux inconvénients des couches-culottes connues grâce à l'utilisation de moyens élastiques de ceinture conçus de manière à améliorer le confort et la capacité d'ajustement de la couche-culotte, cette couche-culotte étant par ailleurs d'un aspect plaisant et d'une fabrication simple. L'invention a également pour objet une couche-culotte avec des moyens élastiques de ceinture conçus de manière qu'ils soient désolidarisés de façon optimale de la partie restante de la couche-culotte. Par ailleurs, l'invention a pour objet une couche-culotte comprenant des moyens élastiques de ceinture conçus de manière à améliorer l'étanchéité de la couche-culotte.

La couche-culotte conforme à l'invention comprend une feuille extérieure imperméable aux liquides, une feuille intérieure perméable aux liquides, les deux feuilles ayant une forme générale sensiblement rectangulaire, un coussin absorbant disposé entre les deux feuilles, le coussin ayant des dimensions inférieures à celles des deux feuilles et étant disposé de manière que ses bords se trouvent en retrait par rapport aux bords correspondants desdites deux feuilles, lesdites deux feuilles étant reliées entre elles par collage le long de leurs bords, autour de coussin absorbant, des attaches adhésives disposées sur les deux bords longitudinaux opposés des feuilles, au voisinage de l'un des deux bords transversaux opposés desdites feuilles, et un moyen élastique de ceinture s'étendant à l'état tendu le long de l'un au moins desdits deux bords transversaux desdites feuilles. Selon l'invention, ledit moyen élastique de ceinture comprend un ruban de support allongé portant au moins un élément élastique tendu s'étendant sensiblement sur toute la longueur dudit ruban. Ledit ruban de support est disposé sur la face interne de la feuille intérieure de la couche-culotte et s'étend sensiblement sur toute la largeur de la couche-culotte de manière que l'élément élast ique se trouve sensiblement en alignement avec les attaches adhésives de la couche-culotte. Ledit ruban de support est fixé à la feuille intérieure de la couche-culotte de manière qu'il soit désolidarisé de ladite feuille au moins à l'endroit dudit élément élastique.

Dans le cadre de l'invention, le ruban de support peut être fixé à la feuille intérieure de la couche-culotte, soit uniquement à ses deux extrémités, soit en plus le long de son bord longitudinal adjacent au bord transversal de la couche-culotte.

Le ruban de support du moyen élastique de ceinture conforme à l'invention peut porter, soit un élément élastique en forme de ruban, soit plusieurs éléments élastiques en forme de brins.

Le ou les éléments élastiques sont de préférence fixés sur le ruban de support sur la face de ce dernier tournée vers la feuille intérieure de la couche-culotte.

Dans la mesure où les couches-culottes sont généralement fabriquées en continu dans le sens de la longueur, en raison de la présence des éléments élastiques longitudinaux d'entre-jambes qui, après leur encollage par intervalles, sont posés à l'état tendu puis sectionnés (procédé dit "snap-back"), la pose de moyens élastiques transversaux à l'état tendu est pratiquement impossible au cours de cette fabrication en continu. C'est la raison pour laquelle, dans le cadre de l'invention, les éléments élastiques des moyens élastiques de ceinture sont avantageusement formés par un matériau élastique du type thermorétractable ou thermo-activable. De tels moyens élastiques peuvent être fixés à l'état détendu sur la feuille intérieure perméable, au cours de la fabrication en continu des couches-culottes, et subissent ensuite, sur les couches-culottes individuelles, un traitement thermique en vue de leur contraction et leur activation.

En se référant aux dessins schématiques annexés, on va décrire ci-après plus en détail plusieurs modes de réalisation illustratifs et non limitatifs d'une couche-culotte conforme à l'invention; sur les dessins :
la figure 1 est une vue en plan sur la face interne d'une couche-culotte comportant des moyens élastiques de ceinture classiques;
la figure 2 est une coupe, à plus grande échelle, suivant II-II de la figure 1;
la figure 3 est une vue en plan sur la face interne d'une couche-culotte suivant un premier mode de réalisation de l'invention;
la figure 4 est une coupe, à plus grande échelle, suivant IV-IV de la figure 3;
la figure 5 est une vue en perspective sur la face interne d'une couche-culotte suivant un deuxième mode de réalisation de l'invention;
la figure 6 est une coupe, à plus grande échelle, suivant VI-VI de la figure 5;
la figure 7 est une vue partielle en perspective d'une variante de la couche-culotte suivant la figure 5.

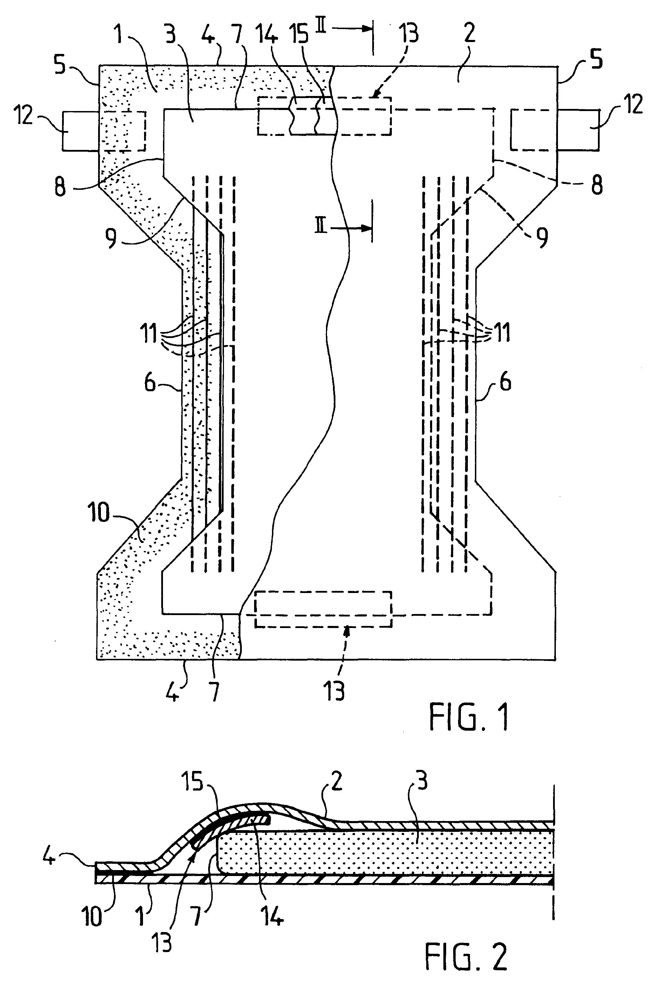

La couche-culotte telle qu'illustrée par les figures 1 et 2 comprend une feuille extérieure 1 imperméable aux liquides, par exemple une feuille de polyéthylène, une feuille intérieure 2 perméable aux liquides, par exemple une feuille de non-tissé, et un coussin ou matelas absorbant 3, par exemple en fibres de cellulose obtenues de préférence par défibrage d'une pâte "fluff" de cellulose, éventuellement avec addition de particules de superabsorbant. Les deux feuilles 1 et 2 présentent une forme générale rectangulaire, leurs deux bords opposés 4 transversaux étant rectilignes, tandis que leurs deux bords opposés 5 longitudinaux sont munis chacun d'une échancrure 6 sensiblement médiane, ce qui donne aux deux feuilles 1 et 2 une forme en sablier.

De façon analogue, le coussin absorbant 3 de forme générale rectangulaire présente deux bords opposés transversaux 7 rectilignes et deux bords opposés longitudinaux 8 munis chacun d'une échancrure 9 sensiblement médiane donnant au coussin 3 une forme en sablier. Le coussin absorbant 3 est plus petit que les deux feuilles 1 et 2 et est disposé en position centrale par rapport aux deux feuilles 1 et 2, de sorte que ses bords 7 et 8 avec les échancrures 9 se trouvent en retrait vers l'intérieur par rapport aux bords 4 et 5 avec les échancrures 6 des deux feuilles 1 et 2.

Les deux feuilles 1 et 2 sont reliées entre elles tout autour du coussin absorbant 3 par collage, comme indiqué en 10.

La couche-culotte est par ailleurs munie de façon usuelle, de deux éléments élastiques longitudinaux 11 constitués, dans l'exemple représenté, chacun par quatre brins élastiques paralleles fixés à l'état tendu à la feuille extérieure 1 de manière à s'étendre le long de l'un de deux bords longitudinaux 8 du coussin 3, dans la zone de l'échancrure 9.

Par ailleurs, la couche-culotte est munie, de façon connue en soi, de deux attaches adhésives 12 fixées au moins à la feuille extérieure 1, sur les deux bords longitudinaux 5, au voisinage de l'un des deux bords transversaux 4, en vue de la fermeture de la couche-culotte autour du corps de l'utilisateur.

La couche-culotte comporte de plus deux moyens élastiques de ceinture 13 au voisinage des deux bords transversaux 4. Chaque moyen élastique de ceinture 13 est constitué par un ruban élastique 14 fixé à l'aide d'une couche de colle 15 (ou par thermoscellage) sur la face externe de la feuille intérieure 2, tournée vers la feuille extérieure 1, le long d'un des bords transversaux 4, en étant décalé par rapport audit bord 4 de manière à se trouver en dehors de la zone de collage 10 entre les deux feuilles 1 et 2. Le moyen élastique de ceinture 13 s'étendant le long du bord transversal 4 au voisinage duquel se trouvent les attaches adhésives 12 est ainsi situé sensiblement en alignement avec les deux attaches 12.

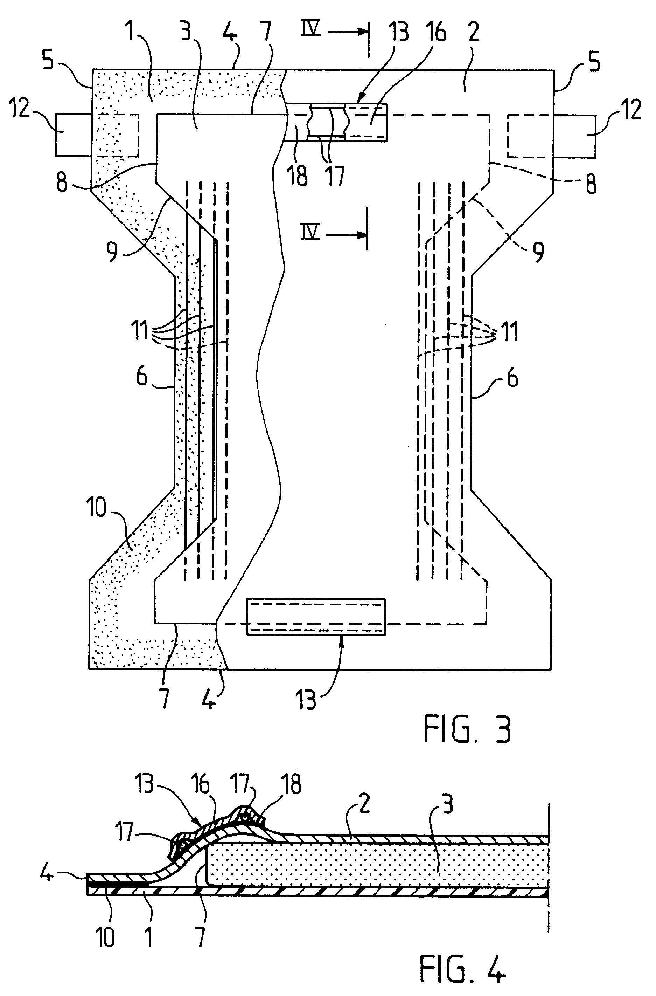

La couche-culotte illustrée par les figures 3 et 4 présente la même structure générale et la même forme générale que la couche-culotte suivant les figures 1 et 2, avec deux feuilles 1 et 2 en forme sablier, reliées entre elles par collage en 10 autour d'un coussin absorbant 3 également en forme de sablier, avec deux éléments élastiques longitudinaux 11 s'étendant le long des deux bords longitudinaux opposés 8 du coussin 3, dans la zone des échancrures 9, et avec deux attaches adhésives 12 disposées sur les deux bords longitudinaux 5 des feuilles 1 et 2, au voisinage d'un des deux bords transversaux 4 desdites feuilles.

Les différences principales, par rapport au mode de réalisation des figures 1 et 2, consistent dans le mode de fixation et dans la nature des moyens élastiques de ceinture 13.

Tout d'abord, les moyens élastiques de ceinture 13 sont ici fixés sur la face interne de la feuille intérieure 2 perméable aux liquides, c'est-à-dire sur la face qui est éloignée de la feuille extérieure 1 et qui est donc tournée vers la peau de l'utilisateur de la couche-culotte. De plus, chaque moyen élastique de ceinture 13, au lieu d'être constitué par un ruban de matière élastique, est formé d'un ruban de support 16 non élastique portant plusieurs brins élastiques 17 parallèles, à savoir au nombre de deux, le ruban composite ainsi formé étant fixé par une couche de colle 18 sur la feuille intérieure 2 perméable.

Les éléments élastiques de ceinture composites selon les figures 3 et 4 peuvent être constitués d'un ruban de support 16 imperméable, par exemple en feuille de polyéthylène ou en non-tissé de préférence hydrophobe, mais il serait également possible d'utiliser un élément élastique composite comprenant par exemple un ruban de mousse de polyuréthane d'une épaisseur comprise par exemple entre 1 et 2 mm, dont une face est encollée avec un ruban imperméable, par exemple en feuille de polyéthylène d'une épaisseur comprise par exemple entre 15 et 20 µm, l'autre face portant des brins élastiques.

Par ailleurs, chaque élément de ceinture élastique 13 disposé au milieu de la largeur de la couche-culotte pourrait être remplacé par deux éléments de ceinture élastique alignés, espacés, disposés plus près des bords longitudinaux 5 de la couche-culotte.

Le nombre des brins élastiques 17 des moyens élastiques de ceinture 13 peut également être différent de deux, en fonction de la largeur des moyens élastiques de ceinture. Il convient de rappeler à ce sujet qu'il est essentiel que le moyen élastique de ceinture situé du côté des attaches adhésives 12 soit disposé le plus possible en alignement avec lesdites attaches 12. Lorsqu'un élément élastique de ceinture 13 est également prévu à l'autre extrémité de la couche-culotte, il doit être disposé de manière à se trouver sensiblement en alignement avec les zones de réception des attaches adhésives 12 après fermeture de la couche-culotte autour du corps de l'utilisateur.

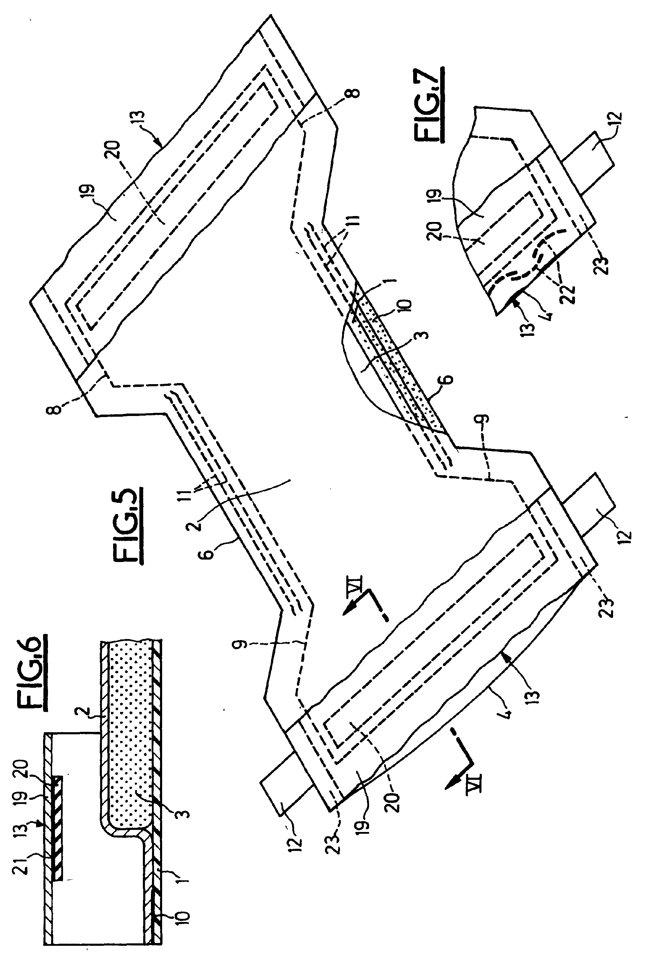

Le mode de réalisation suivant les figures 5 et 6 diffère du mode de réalisation précédent par la structure, la disposition, la longueur et le mode de fixation des moyens élastiques de ceinture.

La couche-culotte illustrée par les figures 5 et 6 présente la même structure générale et la même forme générale que la couche-culotte suivant les figures précédentes, avec deux feuilles 1 et 2 en forme de sablier, reliées entre elles par collage en 10 autour d'un coussin absorbant 3 également en forme de sablier, avec deux éléments élastiques longitudinaux 11 s'étendant le long des deux bords longitudinaux opposés 8 du coussin, dans la zone des échancrures 9, et avec deux attaches adhésives 12 disposées sur les deux bords longitudinaux 5 des feuilles 1 et 2 au voisinage d'un des deux bords transversaux 4.

Les moyens élastiques de ceinture 13 sont ici constitués chacun par un ruban de support 19, par exemple en non-tissé hydrophobe, sur lequel un ruban élastique 20 est fixé à l'aide d'une couche de colle 21. Le ruban 19 présente une longueur correspondant à la largeur de la couche-culotte dans la zone des bords transversaux 4. Le ruban élastique 20 fixé sur le ruban 19 présente une longueur légèrement inférieure à celle du ruban 19. Le ruban 19 portant le ruban élastique 20 est disposé le long du bord 4 proche des attaches adhésives 12, sur la face interne de la feuille intérieure 2, de manière que le ruban élastique 20 se trouve aligné avec ces attaches. Le long de l'autre bord transversal, le ruban de support 19 portant le ruban élastique 20 est disposé de manière que le ruban élastique 20 soit aligné avec les zones de réception des attaches adhésives 12.

Le ruban 19 est disposé sur la feuille 2 de manière que le ruban élastique 20 soit tourné vers la feuille 2 et est fixé, uniquement à ses deux extrémités, par deux zones de fixation 23, par exemple par collage (hot melt) ou thermoscellage, sur la feuille intérieure 2 de la couche-culotte. Par conséquent, chaque ruban 19 portant un ruban élastique 20 forme, entre ses deux extrémités, un moyen élastique de ceinture "flottant", ce qui donne à chaque moyen élastique de ceinture 13 une indépendance maximale par rapport à la couche-culotte proprement dite. Cette indépendance est illustrée sur la figure 5 et 6 par le fait qu'entre ses deux extrémités, le moyen élastique de ceinture 13 est représenté comme étant séparé de la couche-culotte proprement dite, c'est-à-dire disposé à distance au-dessus de la feuille intérieure 2 de la couche-culotte.

Dans la variante suivant la figure 7, le ruban de support 19 portant le ruban élastique 20 est fixé à la feuille intérieure 2 de la couche-culotte, non seulement en 23 à ses deux extrémités, mais également, sur toute sa longueur, le long de son bord longitudinal extérieur adjacent au bord transversal 4 correspondant de la couche-culotte par une ou plusieurs lignes continues ou discontinues de thermoscellage, par exemple une succession de segments de thermoscellage 22, comme représenté sur la figure 7. Dans ce cas, le moyen élastique de ceinture 13 présente une indépendance plus limitée par rapport à la feuille intérieure 2 de la couche-culotte, mais forme simultanément, à l'extrémité de la couche-culotte, c'est-à-dire à la ceinture, une poche améliorant l'étanchéité à cet endroit.

Il y a également lieu de noter que le moyen élastique de ceinture 13 suivant les figures 5, 6 et 7 peut, en cas de besoin, servir au maintien d'une couche absorbante supplémentaire dont les deux extrémités pourront être glissées en dessous des moyens élastiques de ceinture 13, du fait que les rubans 19 sont fixés à la partie restante de la couche-culotte uniquement aux deux extrémités, en 23, et éventuellement le long du bord extérieur, en 22 (selon la figure 7), tandis que les rubans 19 sont libres dans leur partie restante et en particulier à l'endroit des rubans élastiques 20.

Dans un exemple de réalisation préféré, les rubans de support 19 présentent une largeur de l'ordre de 75 mm et portent, au milieu de leur largeur, un ruban élastique 20 d'une largeur de l'ordre de 25 mm.

Les rubans de support 19 qui entrent en contact avec la peau de l'utilisateur sont de préférence constitués par un matériau doux tel qu'un non-tissé qui peut être de préférence hydrophobe.

Dans tous les modes de réalisation représentés et décrits, les éléments élastiques des moyens élastiques de ceinture (ruban 14 ou 20), brins 17) peuvent être constitués par une matière élastique usuelle, par exemple du caoutchouc naturel ou synthétique, auquel cas il est nécessaire de fixer les moyens élastiques à l'état tendu sur la couche-culotte. Les brins élastiques peuvent, par exemple, être constitués par des fils en fibres d'élastane, commercialisés sous la marque "LYCRA XA" par la Société DU PONT.

Cependant, pour simplifier la fabrication des couches-culottes, il est avantageux d'utiliser, pour les moyens élastiques de ceinture, un matériau élastique thermorétractable. En effet, lorsque les moyens élastiques de ceinture sont réalisés à partir de tels matériaux thermorétractables, ils peuvent être fixés à l'état détendu sur la couche-culotte et être soumis, après leur fixation sur la couche-culotte, à un traitement thermique en vue de leur contraction. De tels matériaux élastiques thermorétractables bien connus dans le domaine des couches-culottes sont par exemple décrits dans les documents FR-A-2 583 620 et FR-A-2 583 621.

Bien entendu, l'invention est applicable non seulement à des couches-culottes en forme de sablier, mais d'une manière générale à toutes les couches-culottes et articles d'hygiène analogues pour enfants et pour adultes incontinents.

## Revendications

1. Couche-culotte comprenant une feuille extérieure (1) imperméable aux liquides, une feuille intérieure (2) perméable aux liquides, les deux feuilles ayant une forme générale sensiblement rectangulaire, un coussin absorbant (3) disposé entre les deux feuilles, le coussin ayant des dimensions inférieures à celles des deux feuilles et étant disposé de manière que ses bords (7, 8, 9) se trouvent en retrait par rapport aux bords correspondants (4, 5, 6) desdites deux feuilles, lesdites deux feuilles étant reliées entre elles par collage (10) le long de leurs bords, autour du coussin absorbant, des attaches adhésives (12) disposées sur les deux bords longitudinaux opposés (5) des feuilles, au voisinage de l'un des deux bords transversaux opposés (4) desdites feuilles, et un moyen élastique de ceinture (13) s'étendant à l'état tendu le long de l'un au moins des deux bords transversaux desdites feuilles, caractérisée par le fait que ledit moyen élastique de ceinture (13) comprend un ruban de support (19) allongé portant au moins un élément élastique (20) tendu s'étendant sensiblement sur toute la longueur dudit ruban, que ledit ruban de support (19) est disposé sur la face interne de la feuille intérieure (2) de la couche-culotte et s'étend sensiblement sur toute la largeur de la couche-culotte, de manière que l'élément élastique (20) se trouve sensiblement en alignement avec les attaches adhésives (12) de la couche-culotte, et que ledit ruban de support (19) est fixé à la feuille intérieure (2) de la couche-culotte de manière qu'il soit désolidarisé de ladite feuille (2) au moins à l'endroit dudit élément élastique (20).

2. Couche-culotte suivant la revendication 1, caractérisée par le fait que ledit ruban de support (19) porte un ou plusieurs brins élastiques.

3. Couche-culotte suivant la revendication 1, caractérisée par le fait que ledit ruban de support (19) porte un ruban élastique (20).

4. Couche-culotte suivant l'une quelconque des revendications 1 à 3, caractérisée par le fait que ledit élément élastique est constitué par une matière élastique thermorétractable.

5. Couche-culotte suivant l'une quelconque des revendications précédentes, caractérisée par le fait que ledit ruban de support (19) est fixé à ses deux extrémités (en 23) à la feuille intérieure (2) de la couche-culotte.

6. Couche-culotte suivant la revendication 5, caractérisée par le fait que ledit ruban de support (19) est en outre fixé le long de son bord longitudinal extérieur (en 22) à la feuille intérieure (2) de la couche-culotte.

7. Couche-culotte suivant l'une quelconque des revendications précédentes, caractérisée par le fait que ledit ruban de support (19) est constitué par un non-tissé, de préférence hydrophobe.

8. Couche-culotte suivant l'une quelconque des revendications précédentes, caractérisée par le fait que le ruban de support (19) est fixé à la feuille intérieure (2) de la couche-culotte par collage (hot melt).

9. Couche-culotte suivant l'une quelconque des revendications 1 à 7, caractérisée par le fait que le ruban de support (19) est fixé à la feuille intérieure (2) de la couche-culotte par thermoscellage.

## Patentansprüche

1. Windelhöschen mit einer für Flüssigkeiten undurchlässigen äußeren Folie (1); einer für Flüssigkeiten durchlässigen inneren Folie (2); wobei die beiden Folien allgemein eine im wesentlichen rechteckige Form besitzen; einem zwischen den beiden Folien angebrachten absorbierenden Kissen (3); wobei das Kissen Abmessungen besitzt, die kleiner als die der beiden Folien sind, und wobei es so angebracht ist, daß sich seine Ränder (7, 8, 9) relativ zu den entsprechenden Rändern (4, 5, 6) der beiden Folien in zurückgezogener Lage befinden; wobei die beiden Folien miteinander durch Klebung (10) entlang ihrer Ränder um das absorbierende Kissen herum verbunden sind; Hafthaltern (12), die an den beiden einander gegenüberliegenden Längsrändern (5) der Folien in der Nähe eines der beiden einander gegenüberliegenden Querränder (4) der Folien angeordnet sind; und einem elastischen Gürtungsmittel (13), das sich im gespannten Zustand entlang mindestens eines der beiden Querränder der Folien erstreckt,
dadurch **gekennzeichnet,** daß das elastische Gürtungsmittel (13) ein langgestrecktes Halteband (19) aufweist, das mindestens ein gespanntes, elastisches Element (20) trägt, das sich im wesentlichen über die gesamte Länge des Bandes erstreckt; daß das Halteband (19) auf der Innenfläche der äußeren Folie (2) des Windelhöschens angeordnet ist und sich im wesentlichen über die gesamte Breite des Windelhöschen erstreckt, derart, daß sich das elastische Element (20) im wesentlichen in einer Flucht mit den Hafthaltern (12) des Windelhöschens befindet; und daß das Halteband (19) an der inneren Folie (2) des Windelhöschens so befestigt ist, daß es von der genannten Folie (2) mindestens an der Stelle des elastischen Elementes (20) getrennt ist.

2. Windelhöschen nach Anspruch 1,
dadurch **gekennzeichnet,** daß das Halteband (19) ein oder mehrere elastische Litzen trägt.

3. Windelhöschen nach Anspruch 1,
dadurch **gekennzeichnet,** daß das Halteband (19) ein elastisches Band (20) trägt.

4. Windelhöschen nach einem beliebigen Anspruch 1 bis 3,
dadurch **gekennzeichnet,** daß das elastische Element aus einem sich durch Wärme einziehenden elastischen Material besteht.

5. Windelhöschen nach einem beliebigen vorhergehenden Anspruch,
dadurch **gekennzeichnet,** daß das Halteband (19) an seinen beiden Enden (bei 23) an der inneren Folie (2) des Windelhöschens befestigt ist.

6. Windelhöschen nach Anspruch 5,
dadurch **gekennzeichnet,** daß das Halteband (19) außerdem entlang seines äußeren Längsrandes (bei 22) an der inneren Folie (2) des Windelhöschens befestigt ist.

7. Windelhöschen nach einem beliebigen vorhergehenden Anspruch,
dadurch **gekennzeichnet,** daß das Halteband (19) aus einem, vorzugsweise wasserabweisenden Non-woven besteht.

8. Windelhöschen nach einem beliebigen vorhergehenden Anspruch,
dadurch **gekennzeichnet,** daß das Halteband (19) an der inneren Folie (2) des Windelhöschens durch Klebung (hot melt) befestigt ist.

9. Windelhöschen nach einem beliebigen Anspruch 1 bis 7,
dadurch **gekennzeichnet,** daß das Halteband (19) an der inneren Folie (2) des Windelhöschens durch Heißsiegeln befestigt ist.

## Claims

1. Diaper comprising a liquid-impervious outer sheet (1), a liquid-permeable inner sheet (2), the two sheets being of substantially rectangular overall shape, an absorbent pad (3) arranged between the said two sheets, the pad having dimensions smaller than those of the two sheets and being arranged so that its edges (7, 8, 9) are set back in relation to the corresponding edges (4, 5, 6) of the said two sheets, the said two sheets being joined together by adhesive bonding (10) along their edges, around the absorbent pad, adhesive fastenings (12) arranged on the two opposed lengthwise edges (5) of the sheets, in the vicinity of one of the two opposed transverse edges (4) of the said sheets, and an elastic waistband means (13) extending in the stretched state along at least one of the two transverse edges of the said sheets, characterized in that the said elastic waistband means (13) comprises an elongate supporting strip (19) carrying at least one stretched elastic member (20) extending substantially over the whole length of the said strip, that the said supporting strip (19) is arranged on the inner face of the inner sheet (2) of the diaper and extends substantially over the whole width of the diaper, so that the elastic member (20) is substantially aligned with the adhesive fastenings (12) of the diaper, and that the said supporting strip (19) is secured to the inner sheet (2) of the diaper so that it is detached from the said sheet (2) at least at the place of the said elastic member (20).

2. Diaper according to Claim 1, characterized in that the said supporting strip (19) carries one or a number of elastic strands.

3. Diaper according to Claim 1, characterized in that said supporting strip (19) carries an elastic strip (20).

4. Diaper according to any one of Claims 1 to 3, characterized in that the said elastic member consists of a heat-shrinkable elastic material.

5. Diaper according to any one of the preceding claims, characterized in that the said supporting strip (19) is secured at its two ends (at 23) to the inner sheet (2) of the diaper.

6. Diaper according to Claim 5, characterized in that the said supporting strip (19) is additionally secured along its outer lengthwise edge (at 22) to the inner sheet (2) of the diaper.

7. Diaper according to any one of the preceding claims, characterized in that the said supporting strip (19) consists of a preferably hydrophobic nonwoven.

8. Diaper according to any one of the preceding claims, characterized in that the supporting strip (19) is secured to the inner sheet (2) of the diaper by adhesive (hot melt) bonding.

9. Diaper according to any one of Claims 1 to 7, characterized in that the supporting strip (19) is secured to the inner sheet (2) of the diaper by heat-sealing.
